# EUROPEAN PATENT APPLICATION

(11) **EP 4 484 954 A1**
(43) Date of publication of application: **01.01.2025**
(21) Application number: 23182546.4
(22) Date of filing: 29.06.2023
(51) Int. Cl.: G01N 33/569, A61K 39/00

(54) **MHC CLASS I RESTRICTED IMMUNOPEPTIDE PATTERN FOR GENERATION OF ANTI-NSCLC VACCINES**

(71) Applicant: Uniwersytet Gdanski, 80-309 Gdansk (PL)
(72) Inventor: Kote, Sachin, 80-116 Gdansk (PL); Marek-Trzonkowska, Natalia, 80-752 Gdansk (PL); Pirog, Artur, 80-312 Gdansk (PL); Faktor, Jakub, 80-170 Gdansk (PL); Czaplewska, Pauina, 80-461 Gdansk (PL)
(74) Representative: Czarnik, Maciej

(57) **Abstract**

The invention relates to 2 tissue MHC class I immunopeptide patterns dedicated for generation of anti-cancer vaccines against Non-Small Cell Lung Cancer (NSCLC), identification and isolation of NSCLC specific T cells in humans by antibody-free approach that defined using a list of the 25 quantitative peptides that in NSCLC tumor tissue of patient with healthy tissues are up or down regulated between the comparison of healthy tissue with matched NSCLC patient-derived tissue samples. Therefore, this invention has significant implications for understanding fundamental and applied biological questions.

## Description

The invention refers to tissue MHC class I immunopeptides divided into two patterns dedicated for generation of anti-cancer vaccines against Non-Small Cell Lung Cancer (NSCLC), identification and isolation of NSCLC specific T cells by antibody-free approach.

Presentation of major histocompatibility complex (MHC) ligands on the cell surface is the essential way of communicating the state of the cell to the immune system, then immune recognition and activation. Any disturbances in this process may be a potential cause of disease. For example, autoimmune disorders may emerge when the immune system incorrectly recognizes cells as non-self and attacks them. On the other hand, cancer cells may hide from immunity when no signal on the cell surface can provide an impulse for the immune system to kill the altered cells. The identification and characterization of peptides presented on the surface of cells in complex with major histocompatibility complex (MHC) molecules is also called immunopeptidomics analysis. Moreover, developing a method for detecting mutated or non-canonical peptide precursors derived from patient samples, such as tumor tissue, and presented by histocompatibility antigens (MHC) is highly demanded. Moreover, determining the peptide presentation pathway from the translation product to the MHC ligand in various disease scenarios will be necessary for developing vaccines, autoimmune disease therapies, cellular therapies, and anticancer and antiviral therapies. Therefore, analysis of immunopeptidomics provides valuable information about the repertoire of peptides. Furthermore, in the context of neoantigen discovery and immunotherapy, it is necessary to find the unique antigens on the cancer cell surface, also called neoantigens. The source of neoantigen is from the tumor-specific mutations or aberrantly expressed proteins, making them attractive targets for cancer immunotherapy. Therefore, neoantigen discovery is becoming an active research area and has the potential to revolutionize cancer treatment by harnessing the immune system's power to target and eliminate tumor cells with high specificity. Immunotherapy is a rapidly evolving medical field that has revolutionized the treatment of various diseases. It harnesses the immune system's power to recognize, attack, and destroy abnormal cells, such as cancer cells or cells infected with pathogens. Immunotherapy includes immune checkpoint inhibitors, Chimeric Antigen Receptor T-cell (CAR-T) therapy, cancer vaccines, adoptive cell transfer, etc. Recently, It has shown significant advancements in treating cancer, infectious diseases, autoimmune disorders, and other conditions. Ongoing research and development in immunotherapy are focused on optimizing treatment strategies, expanding the range of treatable diseases, and improving patient outcomes. For the first time, the inventors have proposed Tissue MHC class I immunopeptide pattern dedicated for generation of anti-cancer vaccines against Non-Small Cell Lung Cancer (NSCLC), identification and isolation of NSCLC specific T cells in humans and differential immunopeptides from healthy tissues by antibody-free approach. The invention is simple, cost-effective, fast, and comprehensive for solid tumor MHC class I immunopeptide profiling. The antibody-free approach is available to apply for a wide range of disease models for generation of anti-cancer vaccines against other cancer, identification and isolation of other cancer specific T cells in humans.

Immunoprecipitation (IP) is a widely used method for immunopeptidomics analysis to screen the [1] immunopeptidome. However, this approach has several technical limitations such as a) antibody dependent/biased means due to the binding specificity of the antibody, MHC subtype information is also lost in IP-based methods utilizing the pan MHC antibody [2] b) IP requires extensive washes to remove contaminants/unspecific peptides and detergent, which may lead to losses of low-affinity MHC I-associated peptides [3] [4], c) due to antibody dependence, it become very costly for higher numbers of samples preparation, d) It is time consuming and needs a highly skilled personnels, e) inability to employ for non-human model due to lack of efficient antibodies for other species. The invention includes the direct isolation, qualitative, and quantitative screening of immunopeptidome on the major histocompatibility (MHC) class I molecules from the solid tumor by antibody free approach. Patient-derived tissue immunopeptidome, allowing to screened neoantigens , can serve as a vaccine candidate and develop T cell-based cancer therapies. The invented approach is the comparatively quick, high throughput and cost effective pipeline, globally applicable for direct purification and quantification of neoantigens from the tumor patient samples using the unbiased mass spectrometry analytical tool.

In the contrary, antibody free tissue MHC class I immunopeptidomics approach possesses several advantages. The inventor reveals for the first time the immunopeptidome on the major histocompatibility (MHC) class I molecules from the solid tumor by antibody free approach. This is a simple, economical, and parallel approach that can be used with immunoprecipitation (IP) for neoantigen discovery. Therefore, inventors identify several areas that will significantly profit from the immunopeptidome development of solid tumor by antibody free approach.
1. The potential of an invention is to identify and quantify the MHC class I patient and disease-specific immunopeptidomes. 2. Antibody free approach is unbiased toward any antibody epitope specificity with MHC class I alleles. Therefore, the invention allowing to address the following questions; a) What is the variability and specificity of neoantigens peptidome between control and respective patients derived tissue samples, b) Neoantigen landscape specific to an individual patient, so there is a possibility of developing personalized cancer therapy. 3. Screening and discovery of common neoantigens concerning various cancer types that suites developing global immunotherapy for various kinds of cancer patients. Therefore, new customs will be developed for the entire medical field, e.g., routine screening of neoantigens. 4. The invention approach (antibody free approach) is applicable even though the starting tissue material may be limited (less than 0.5 grams). On the contrary, for immunoprecipitation (IP) base samples preparation is recommended to use at least 1 gram of tissue for obtaining optimal MHC class I peptides yield. 5. The invention approach allows for cell regeneration following β2M-dissociation, i.e., after the first time isolation of MHC class I immunopeptidome, so it can be re-analyzed after a second and subsequent perturbation. Therefore the dissociation kinetics of MHC class I immunopeptidome can be done easily. 6. The antibody free approach is simple, quick, and reproducible. Particularly the sample preparation for qualitative and quantitative tissue on the major histocompatibility (MHC) class I immunopeptidomics analysis by antibody-free approach in mammalian tissue samples. It is based on amino acid sequencing with tandem mass spectrometry with signaling intensities measurement of peptides characterized by only two steps of sample preparation as follows: a) extraction of tissue MHC class I immunopeptidomics by antibody-free approach, b) purification of tissue MHC class I immunopeptidome. 7. The invention approach (antibody free approach) is the only option for the scientific community where antibodies against MHC I molecules are not available, especially for non-human tissue samples (wild boar, cheetah, panther, etc.) to perform the immunopeptidome analysis. 8. The invention involves fewer purification steps, and no detergents are involved during the sample preparation. 9. The invention introduces no bias linked to preferential loss of low/medium/high-affinity peptides recovery. 10. Invention approach has great potential to open numerous branches for various diseases that, many based on immune response and gown in academic, medical, biotechnology, and industrial development. 11. Invention has tremendous potential to be translational, commercialization for high-throughput screening of MHC class I peptides, and subsequently, the antibody-free approach is available to apply for a wide range of disease models for generation of anti-cancer vaccines against other cancer, identification and isolation of various types of clinical tumor samples cancer specific T cells in humans. The invention refers to 25 tissue MHC class I immunopeptide peptides- patterns in total divided on two patterns according to up and down regulated pattern dedicated for generation of anti-cancer vaccines against Non-Small Cell Lung Cancer (NSCLC), identification and isolation of NSCLC specific T cells in humans and differential immunopeptides from healthy tissues by antibody-free approach. The invention is simple, cost-effective, fast, and comprehensive for solid tumor MHC class I immunopeptide profiling.

Tissue MHC class I immunopeptide patterns dedicated for generation of anti-cancer vaccines against Non-Small Cell Lung Cancer (NSCLC), identification and isolation of NSCLC specific T cells in humans that is a list of 25 quantitative MHC class I immunopeptide wherein the MHC class I immunopeptides level of mentioned 25 peptides are up or down regulated between the comparison of healthy tissue with matched NSCLC patient-derived tissue samples. The up and down regulated refers only to MHC class I tissue quantitative peptides; it shows how many times more signaling intensities present in healthy tissue samples were higher than in non-small cell lung cancer (NSCLC) tissue samples. Therefore, all these at least 25 quantitative immunopeptidome, human leukocyte antigen (HLA) binder, and the peptide length between 7-16 amino acids (majority at 9 mers) MHC class I immunopeptidome patterns for lymphocyte-related immune responses (lymphocyte attenuation) in NSCLC. The invention is related to the tissue MHC class I immunopeptide pattern dedicated for generation of anti-cancer vaccines against Non-Small Cell Lung Cancer (NSCLC), identification and isolation of NSCLC specific T cells in humans and differential immunopeptides from healthy tissues by antibody-free approach. The invention is based on the identification (qualitative analysis) and relative quantification (quantitative analysis) of a unique MHC class I immunopeptide pattern dedicated for generation of anti-cancer vaccines against Non-Small Cell Lung Cancer (NSCLC), identification and isolation of NSCLC specific T cells in humans (identified based on amino acid sequences). 25 quantitative MHC class I immunopeptides wherein the MHC class I immunopeptides level of mentioned 25 peptides are up or down regulated between comparing healthy tissue with matched NSCLC patient-derived tissue samples. (quantitative analysis). Dysregulated MHC class I quantitative peptides correlate positively with their predictive significance for generation of anti-cancer vaccines against Non-Small Cell Lung Cancer (NSCLC), identification and isolation of NSCLC specific T cells in humans. Inventors also define tissue MHC class I immunopeptide patterns as upregulated and down regulated MHC class I immunopeptides. The tissue MHC class I immunopeptide patterns pattern of upregulated, wherein healthy tissue MHC class I immunopeptide levels of the 18 quantitative peptides wherein the tissue MHC class I immunopeptide level of mentioned 18 immunopeptides are up-regulated in healthy tissue with matched NSCLC patient-derived tissue samples. The tissue MHC class I immunopeptide patterns pattern of downregulated, wherein healthy tissue MHC class I immunopeptide levels of the 7 quantitative peptides wherein the tissue MHC class I immunopeptide level of mentioned 18 immunopeptides are downregulated in healthy tissue with matched NSCLC patient-derived tissue samples. Each of the up or down regulated tissue MHC class I immunopeptide patterns pattern can be used as a separate independent panel of tissue MHC class I immunopeptide patterns dedicated for generation of anti-cancer vaccines against Non-Small Cell Lung Cancer (NSCLC), identification and isolation of NSCLC specific T cells in humans and neoantigen discovery of NSCLC tumor and differentiation from healthy tissues and further selection for making them attractive targets for cancer immunotherapy.

Tissue MHC class I immunopeptide patterns dedicated for generation of anti-cancer vaccines against Non-Small Cell Lung Cancer (NSCLC), identification and isolation of NSCLC specific T cells in humans. The invention method of tissue MHC class I immunopeptidome by antibody free approach highlights a quantitative evaluation of complex peptide mixture to reveal the diverse nature of tissue MHC class I immunopeptidome. The invention workflow comprehensively, at least 8565 MHC class I peptides were quantitated. Therefore, all these at least 25 quantitative immunopeptidome, HLA binder, and the peptide length between 7-16 amino acids (majority at 9 mers) MHC class I immunopeptidome patterns for lymphocyte-related immune responses (lymphocyte attenuation) in NSCLC. Tissue MHC class I immunopeptide patterns dedicated for generation of anti-cancer vaccines against Non-Small Cell Lung Cancer (NSCLC), identification and isolation of NSCLC specific T cells in humans that is a list of the following 25 quantitative MHC class I immunopeptide Lys-Pro-Ala-Gly-Pro-Pro-Gly-Ile-Leu-Ala-Leu, Tyr-Glu-Phe-Lys-Phe-Pro-Asn-Arg-Leu, Asp-Val-Tyr-Asn-His-Phe-Leu-Leu-Tyr, Phe-Thr-Leu-Gln-Ser-Glu-Leu, Lys-Glu-Asp-Asp-Ala-Pro-Arg-Thr-Ile, Thr-Glu-Phe-Thr-Pro-Thr-Glu-Lys, Phe-Arg-Ile-Gly-Phe-Gly-Ser-Phe, Lys-Thr-Asn-Gly-Lys-Phe-Leu-Ile-Arg, Met-Pro-Val-Gly-Pro-Asp-Ala-Ile-Leu-Arg-Tyr, Gln-Gly-Phe-Pro-Asn-Arg-Val-Val-Phe, Tyr-Val-Tyr-Glu-Tyr-Pro-Ser-Arg-Tyr, Asn-Ala-Ala-Glu-Arg-Arg-Gly-Pro-Leu, Ala-Pro-Arg-Thr-Val-Ala-Leu-Thr-Ala, Met-Pro-Val-Gly-Pro-Asp-Ala-Ile-Leu-Arg-Tyr, Thr-Ile-Ile-Asp-Ile-Leu-Thr-Lys-Arg, Arg-Tyr-Leu-Glu-Lys-Pro-Met-Glu-Ile, Gly-Thr-Tyr-Val-Ser-Ser-Val-Pro-Arg, Gly-Thr-Asp-Pro-Thr-Pro-Gln-His-Tyr, Ala-Ile-Leu-Asp-Glu-Thr-Lys-Gly-Asp-Tyr, Val-Leu-Lys-Thr-Pro-Ser-Ala-Ala-Tyr, Asp-Leu-Val-Pro-Val-Pro-Thr-Asn-Leu, Val-Ile-Glu-Glu-Val-Pro-Gly-Glu-Leu, Tyr-Gln-Lys-Met-Tyr-Gly-Ile-Ser-Leu, Lys-Gly-Asp-Gly-Pro-Val-Gln-Gly-Ile-Ile-Asn-Phe, Ile-Glu-Val-Asp-Asp-Glu-Arg-Lys-Leu-Arg-Thr-Phe wherein the MHC class I immunopeptides level of mentioned 25 peptides are up or down regulated between the comparison of healthy tissue with matched NSCLC patient-derived tissue samples.

The tissue MHC class I immunopeptide pattern, wherein healthy tissue MHC class I immunopeptide levels of the following 18 quantitative peptides Lys-Pro-Ala-Gly-Pro-Pro-Gly-Ile-Leu-Ala-Leu, Tyr-Glu-Phe-Lys-Phe-Pro-Asn-Arg-Leu, Asp-Val-Tyr-Asn-His-Phe-Leu-Leu-Tyr, Phe-Thr-Leu-Gln-Ser-Glu-Leu, Lys-Glu-Asp-Asp-Ala-Pro-Arg-Thr-Ile, Thr-Glu-Phe-Thr-Pro-Thr-Glu-Lys, Phe-Arg-Ile-Gly-Phe-Gly-Ser-Phe, Lys-Thr-Asn-Gly-Lys-Phe-Leu-Ile-Arg, Met-Pro-Val-Gly-Pro-Asp-Ala-Ile-Leu-Arg-Tyr, Gln-Gly-Phe-Pro-Asn-Arg-Val-Val-Phe, Tyr-Val-Tyr-Glu-Tyr-Pro-Ser-Arg-Tyr, Asn-Ala-Ala-Glu-Arg-Arg-Gly-Pro-Leu, Ala-Pro-Arg-Thr-Val-Ala-Leu-Thr-Ala, Met-Pro-Val-Gly-Pro-Asp-Ala-Ile-Leu-Arg-Tyr, Thr-Ile-Ile-Asp-Ile-Leu-Thr-Lys-Arg, Arg-Tyr-Leu-Glu-Lys-Pro-Met-Glu-Ile, Gly-Thr-Tyr-Val-Ser-Ser-Val-Pro-Arg, Gly-Thr-Asp-Pro-Thr-Pro-Gln-His-Tyr wherein the tissue MHC class I immunopeptide level of mentioned 18 immunopeptides are up-regulated in healthy tissue with matched NSCLC patient-derived tissue samples.

The tissue MHC class I immunopeptide pattern, wherein healthy tissue MHC class I immunopeptide levels of the following 7 quantitative peptides Ala-Ile-Leu-Asp-Glu-Thr-Lys-Gly-Asp-Tyr, Val-Leu-Lys-Thr-Pro-Ser-Ala-Ala-Tyr, Asp-Leu-Val-Pro-Val-Pro-Thr-Asn-Leu, Val-Ile-Glu-Glu-Val-Pro-Gly-Glu-Leu, Tyr-Gln-Lys-Met-Tyr-Gly-Ile-Ser-Leu, Lys-Gly-Asp-Gly-Pro-Val-Gln-Gly-Ile-Ile-Asn-Phe, Ile-Glu-Val-Asp-Asp-Glu-Arg-Lys-Leu-Arg-Thr-Phe wherein the tissue MHC class I immunopeptide level of mentioned 7 immunopeptides are down-regulated in healthy tissue with matched NSCLC patient-derived tissue samples.

Each of the up or down regulated tissue MHC class I immunopeptide patterns pattern can be used as a separate independent panel of tissue MHC class I immunopeptide patterns for pattern dedicated for generation of anti-cancer vaccines against Non-Small Cell Lung Cancer (NSCLC), identification and isolation of NSCLC specific T cells in humans and differentiation from healthy tissues and further selection for making them attractive targets for cancer immunotherapy. The up and down regulated refers only to MHC class I tissue quantitative peptides. It shows how many times more signaling intensities present in the tissue of healthy samples were higher than in non-small cell lung cancer (NSCLC) tissue samples. Screening and discovery of common neoantigens concerning various cancer types that suites developing global immunotherapy for various kinds of cancer patients. Therefore, new customs will be developed for the entire medical field, e.g., routine screening of neoantigens so it will revolutionize the early immunotherapy field. Therefore, the invention patient derived tissue samples and MHC class I immunopeptidomics data are complementary to other -omics techniques, such as proteomics. The invention is readily applicable worldwide based on clinical samples like tissue. In addition, the invention approach is comparatively faster (time-wise) and cheaper for directly finding significant biological extracellular tissue peptides pattern dedicated for generation of anti-cancer vaccines against Non-Small Cell Lung Cancer (NSCLC), identification and isolation of NSCLC specific T cells in humans and possible neoantigens from the screened MHC class I immunopeptidomics data from the patient derived tissue samples using the unbiased mass spectrometry analytical tool. The invention opens the way for generation of peptide or mRNA-based vaccines against NSCLC. In terms of peptide vaccines, the identified and quantified by us peptides can be chemically synthesized and delivered into humans (e.g. injection). In terms of mRNAs, the mRNAs encoding our identified peptide sequences can be delivered into human body and then translated to induce immune responses against NSCLC. The most promising approach of the peptide sequences utility is their application in combination with tetramer technology for isolation of peptide specific T cells which can be expanded and used in adoptive therapy against NSCLC.

### Example 1

The invention method is novel, simple, and it has only two steps sample preparation s that provides the screening and quantification of tissue MHC class I immunopeptidome by antibody free approach. Prior to starting any sample preparation steps of the tissue MHC class I immunopeptidome by antibody free method, it is strongly recommended to prepare all the solutions using LC-MS (Liquid Chromatography with mass spectrometry) grade water, solvents, reagents, and ultraclean glass and plasticware use during methods. Moreover, all solutions and buffers should be prepared immediately before use and disposed of after one week of storage. The first step is to prepare all the necessary buffers on the same day of tissue MHC class I immunopeptidome by antibody free method samples preparation.

### Buffer preparations

1^{st} buffer: citrate-phosphate buffer at pH 3.3 - (0.131 M citric acid/0.066 M Na2HPO4, NaCl 150 mM) and adjust the pH 3.3 with 1M NaOH prepared in LC-MS (Liquid Chromatography with mass spectrometry) grade water.
2^{nd} solution, 0.2% formic acid/methanol volume per volume (v/v), was prepared (the final proportion of formic acid in methanol was 0.2%).
3^{rd} solution, 0.2% formic acid/water volume per volume (v/v), was prepared (the final proportion of formic acid in water was 0.2%).
4^{th} solution wash buffer, water/5% methanol/0.2% formic acid volume per volume (v/v), was prepared (the final proportion of methanol and formic acid in water was 5% and 0.2%, respectively).
5^{th} solution elution buffer, water/80% methanol/0.2% formic acid volume per volume (v/v), was prepared (the final proportion of methanol and formic acid in water was 80% and 0.2%, respectively).

Efforts were made to develop a repeatable and effective method to obtain a sufficient amount of sample from the smallest possible amount of tissue material.

Workflow has two steps sample preparation, a) extraction b) purification of tissue peptides.

### a. extraction of tissue MHC class I immunopeptidomics by antibody-free approach

Prior the extraction, the tissue samples are subjected to 7-10 washes with phosphate-buffered saline (PBS) without additives (no calcium/magnesium/phenol red) pH 7.2-7.5 centrifuged at 500 ×g for 5 minutes at 4 °C. Then test tissue samples are incubated with a extraction buffer (citrate-phosphate buffer) with pH 3.3 prepared in liquid chromatography-mass spectrometry (LC-MS) grade ultrapure water, purity ≥ 99.9%, wherein the buffer is at a volume of 5-6ml to 0.5-1 gram of tissue sample. The incubation step takes 4-5 minutes with mixing at a temperature of 4 to 10 °C to dissociate peptides MHC class I of tissue membrane surface are collected by centrifuging at 500 ×g for 5 minutes at 4 °C;

### b. purification of MHC class I immunopeptidome

Collected supernatants for both samples set were subjected to the Oasis cartridges (30 mg; Waters, hydrophilic-lipophilic balanced columns) to perform reverse-phase purification of polypeptides. All steps were performed at room temperature using only gravity flow, and all the detailed steps are as follows.
i) Cartridge's condition: cartridge was conditioned with 0.2% formic acid/methanol volume per volume (v/v) (in this solution final proportion of formic acid in methanol was 0.2%) twice, volume for each wash was 1ml/cartridge.
ii) Equilibration of cartridge: the cartridge was equilibrated with 0.2% formic acid/water volume per volume (v/v) (in this solution final proportion of formic acid in water was 0.2%), volume for each wash was 1ml/cartridge.
iii) Sample loading: 6-8 ml of a sample that this step was the supernatant collected after centrifugation was loaded on the cartridges (hydrophilic-lipophilic balanced columns).
iv) Washing steps: cartridge was washed three times with water containing 5% methanol/0.2% formic acid volume per volume (v/v) (in this solution final proportion of methanol and formic acid in water was 5% and 0.2%, respectively), volume for each wash was 1ml/cartridge.
v) Elution: the bound material (peptides) was eluted with 1ml water containing 80% methanol/0.2% formic acid volume per volume (v/v) (in this solution final proportion of methanol and formic acid in water was 80% and 0.2%, respectively) and then diluted to water/40% methanol/0.2% formic acid volume per volume (v/v) (in this solution final proportion of methanol and formic acid in water was 40% and 0.2%, respectively),

Tissue MHC class I immunopeptidomics, to eliminate β2 microglobulin (B2M) or extracellular material equal or bigger than 3kDa from peptide enriched and further separated with ultrafiltration using selected molecular weight cutoff filter that has 3kDa molecular weight filters for 120-135 minutes centrifuge at 4000 g, at 4 - 10 °C to collect MHC class I peptides smaller than 3kDa. Lastly, the pipette eluted and diluted peptide solution (2ml) into the device, taking care not to touch the membrane with the pipette tip. Collect the filtrate (MHC class I peptides), then evaporate it to dryness. All dried peptide samples were stored at -80 until the mass spectrometry analysis.

### Example 2

Tissue MHC class I immunopeptidome by antibody free approach is simple, economical and parallel approach that can be used with immunoprecipitation (IP) for neoantigen discovery. After the isolation and purification of tissue based MHC class I immunopeptidome by antibody free approach, the next crucial step in the invention is Mass spectrometry-based measurements such as DDA (data-dependent acquisition) and DIA - (data-independent acquisition) mode. Mass spectrometry combined with chromatographic separation is necessary to identify several thousand peptides. Due to the unspecific nature of MHC class I immunopeptidome, successful identification requires the order of magnitude higher data quality than a standard trypsin-based proteomic sample, especially in terms of mass accuracy. Only recently, technological advances in mass spectrometry enabled the collection of data of quality acceptable for such analyses. DIA - (data-independent acquisition) method enables to a collection of quantitative data for all peptides present in the sample while maintaining good resilience to matrix interferences.

### MHC class I immunopeptidome LC-MS/MS (Liquid Chromatography with tandem mass spectrometry) analysis

All dried immunopeptide samples were resuspended in 30 µl loading buffer (0.08% trifluoroacetic acid (TFA) in water with 2.5% acetonitrile (ACN). Each sample was spiked with retention time standard iRT peptides (Biognosys, Switzerland) according to the manufacturer's guidelines. Following, 6 µl of the dissolved sample were injected into a Thermo Scientific, UltiMate^{™} 3000 RSLCnano System (Thermo Scientific, MA, USA) liquid chromatography. Peptides were loaded on an Acclaim^{™} PepMap^{™} 100 C18, 5µm, ID= 1 mm, Length = 5 mm, (Thermo Scientific, MA, USA) trap column using a 5 µl/min loading buffer flow. Subsequently, peptides were separated on PepMap^{™} 100 C18, 5µm, ID= 1 mm, Length = 5 mm, particle size = 2µm (cat. no: 164534) (Thermo Scientific, MA, USA) analytical column. Analytical separation was performed by a non-linear increase of mobile phase B (0.1% formic acid (FA) in ACN (v/v)) in a mobile phase A (0.1% FA in water (v/v)). The non-linear gradient started at 2.5% B, linearly increasing up to 35% B in 180 min, followed by a linear increase up to 60% B in the next 15 min with a constant flow rate of 300 nl/min. Immunopeptides eluting from the column were ionized in a nano-electrospray ion source (NSI) and were introduced to Orbitrap Exploris^{™} 480 Mass Spectrometer (Thermo Scientific, MA, USA) online coupled to the liquid chromatograph.

The data-dependent acquisition (DDA) method, briefly, Exploris 480 acquired the data in data-dependent peptide mode. The full scan was operated in profile mode with 120000 resolution, and the precursor MS scan range was set from m/z 350 Th to m/z 1650 Th. Normalized AGC target was set to 300% with 100 msec maximum injection time. Each MS scan was followed by a fragmentation of the top 20 the most intense precursor ions and the acquisition of their MS/MS spectra. The dynamic mass exclusion was set to 20 sec after the first precursor ion fragmentation. Precursor isotopologues were excluded, and precursor isolation mass tolerance was set to 10 ppm. The minimum precursor ion intensity was set to 3.0e3, and only precursor charge states of +1 to +5 were included in the experiment. The precursor isolation window was set to 1.6 Th. Normalized collision energy type with fixed collision energy mode was selected. The collision energy was set to 30%. Orbitrap resolution was set to 60000. The normalized AGC target was set to 100% with an automatic setting on maximum injection time, and data type was centroid.

The data independent acquisition (DIA) method was used to retrieve quantitative tissue immunopeptidomics data. LC separation parameters were kept identical to DDA acquisition. Orbitrap Exploris 480 mass spectrometer operated in positive polarity data-independent mode (DIA) mode accompanied by a full-scan with 60000 resolution. The full-scan mass range was set from m/z 350 Th up to m/z 1450 Th, and the normalized AGC target was set to 300% with 100 msec maximum injection time. The DIA method cycled in a mass range from m/z 350 Th up to m/z 1100 Th with 12 Th window width and 1 Th overlap. The method performed 62 precursor windows/scan events per one cycle. Normalized collision energy type with fixed collision energy mode was selected. The collision energy was set to 30% and orbitrap resolution to 30000. Normalized AGC target was set to 1000% with the automatic setting of maximum injection time. Data type was profile.

### Data analysis of MHC class I immunopeptidome

Qualitative tissue MHC class I immunopeptidome eluate analysis and spectral library generation; a multi-search engine strategy has been employed to identify immunopeptides in both DDA and DIA MS data. Raw DDA files were centroided and converted into mzML and mzXML format using MSconvert version: 3.0.19094. DIA raw files were directly processed in DIA Umpire SE embedded in FragPipe (v.15). Extracted pseudo-DDA data were converted to centroided mzML and mzXML format. mzML files were searched using MSFragger 3.4 and mzXML files using Comet (Release 2020.01, revision 2) engines against Homo sapiens SwissProt+UniProt and iRT (Biognosys, Switzerland) search database (12_2022). The target search database was concatenated with a reverse decoy database containing reversed target sequences of targets and common contaminant protein sequences. The following search settings were used for MSFragger: precursor mass tolerance was set to +-8 ppm and fragment mass tolerance to 10 ppm. Enzyme digestion was set to non-specific and peptide length was set from 7 to 45 amino acids. Peptide mass range was set from 200 to 5000 Da. Variable modifications were set to: methionine oxidation and protein N-term acetylation. Data were mass recalibrated, and automatic parameter optimization setting was used to tune fragment mass tolerance. The output file format was set to pep.XML. Comet precursor mass tolerance was set to 8 ppm and fragment mass tolerance to 8 ppm. The rest of the settings were identical to MSFragger. The search results (pep.XML) files were processed with PeptideProphet and iProphet as part of TPP 5.2.0. The peptide identifications in retrieved pep.XML files were further processed in R for visualization and visualized in ggplot2 and eulerr 6.1.1. R packages. The pep.XML files with recalculated peptide iprobabilities were further processed in Skyline-daily (64-bit, 20.1.9.234) to a spectral library. A blank document in Skyline-daily was set prior to loading pep.XML files. In "Peptide settings," a "Library" tab was selected. An option of "Build" was selected and in the newly popped-up window the "Cut-off score" was set to 0.99 and as an iRT standard peptides were selected "Biognosys-11 (iRT-C18)". All pep.XML files were loaded. Next, in the "Digestion" tab in the "Background proteome" section the "add" option was selected. In the newly popped up window the background proteome was created from the .FASTA file that was previously used as a search library. Next, in the "Library" tab, "Libraries" section the newly built spectral library was selected and the "Explore" button was clicked to open the library. In the library window an "Associate proteins" option was checked and the "Add all" button was clicked. The retention time calculator was automatically generated based on the iRT peptide retention time values observed in library pep.XML files. Such a prepared Skyline file was saved on the PC hard disc drive.

### Data-Independent Acquisition (DIA) data extraction

DIA data extraction was performed using Skyline-daily (64-bit, 20.1.9.234) with spectral library and document generated in the previous step. In the "Peptide settings" tab, the "Max. missed cleavages" was set to 0. Next, in the "Filter" tab, a maximum peptide length was set from 4 to 200 amino acids. The "Exclude N terminal AAs" option was set to zero. A function of the "Auto-select all matching peptides" was selected. In the "Modifications" tab, no modifications were selected. In the "Library" tab, in the "Libraries" window, a spectral library that was created in the previous step was selected. Other settings in the tab were left default. In transition settings, the "Prediction" tab was left default. In the "Filter" tab, +1, +2, +3, +5, +6 peptide precursor charges were specified. Ion charges were set to +1 and +2. Ion type was set to y and b. Product ion selection was set as follows: in the "From:" window the "ion 4" and in the "To:" window the "last ion" options were selected. The "Auto select all matching transition" option was selected at the bottom of the tab and in the "Special ions:" menu the "N-terminal to Proline" option was selected. In the "Library" tab, the "Ion match tolerance" window was set to 0.05 m/z and only peptides that have at least 4 product ions were kept in analysis. In addition, if more product ions were available per peptide, then the 6 most intense were picked up from the filtered product ions. A function of the "From filtered ion charges and types" was chosen. In the "Instrument" tab inventors included product ions from m/z 350 up to m/z 1100. The "Method match tolerance m/z" window was set to 0.055 m/z mass tolerance. MS1 filtering was set to none in the full scan tab and in MS/MS filtering section, the "Acquisition method" window the "DIA" option was selected and in the "Product mass analyzer," the "Orbitrap" option was selected. In the "Isolation scheme:" the "Add" option was selected and in the "Edit isolation scheme" pop-up window an option of the "Prespecified isolation windows" was selected. The "Import" isolation windows from DIA raw file option was then selected and the isolation scheme was read from a DIA raw file. Back in the "Full-Scan" tab in the "Resolving power:" section the resolving power was set to 30000 at 200 m/z. In the retention time filtering section, "Use only scans within 5 minutes of MS/MS IDs" was specified. Under the "Refine" tab, the "Document" section of the "Advanced" window the "Min transitions per precursor" option was set to 4. Empty proteins were removed from the document. An equal number of reverse sequence decoys via the "Add Decoys" function in the "Refine" tab was added. In the "Add Decoy Peptides" pop-up window a reverse sequence decoy generation method to create decoy peptides was selected. Following, DIA raw files were imported. mProphet model to reintegrate peptide boundaries was trained as follows: in the "Refine" tab the "Reintegrate" function was selected. In the "Reintegrate" pop-up window in the "Peak scoring model:" the "Add" option was selected. Following, in the "Edit Peak Scoring Model," mProphet model was activated. mProphet model was trained using targets and decoys. The "Score rows" with negative "Weight" and/or "Percentage Contribution" (red highlighted) were removed, and the mProphet model was then re-trained. New mProphet peak scoring model was then selected in the "Edit peak scoring Model" window and applied for reintegration of new peak boundaries. Export report function to export a summary of all dependencies required for MSstats 4.0.1. was used to generate quantitative report for downstream analysis.

### Data-Independent Acquisition (DIA) statistical data analysis in MSstats

Statistical analysis of Skyline extracted DIA data was performed in R (version 4.1.2) package MSstats 4.0.1. The protein column was combined with the peptide sequence column to preserve analysis at peptide level, this prevents summing peptide intensities originating from one protein. Extracted peakgroups were reduced by filtering on mProphet q-value 0.01 cut-off. SkylinetoMSstatsFormat function was set to keep proteins with one feature and to transform Skyline output to MSstats input. Further, peptide intensities were log2 transformed and quantile normalized. Differential immunopeptide quantitation across specified conditions was performed pairwise via mixed-effect models implemented in the MSstats "groupComparison" function. p-values were adjusted using the Benjamini-Hochberg method and output result matrix was exported for downstream analyses. Volcano plots were generated in EnhancedVolcano 1.10.0, venn diagrams in eulerr 6.1.1. package, bar graphs in plyr 1.8.6. and ggplot2 3.3.5. all running under R (version 4.1.2).

### Tissue MHC class I immunopeptidome predictions

To predict the binding affinities of peptide sequences to a set of alleles with a frequency not lower than 6.4%, inventors used the MHCflurry package (version 3.1.0) in Python 3.9.13. This package utilizes a pre-trained deep learning model and a large-scale dataset of peptide-MHC binding affinities. Peptides with predicted affinity below 50 were classified as strong binders, peptides with affinity between 50 and 150 as mid-binders, peptides with affinity between 150 and 500 as weak binders, and peptides with affinity above 500 as non-binders.
The allele-independent MHCflurry predictions utilized deep learning architectures such as convolutional neural networks (CNNs) or recurrent neural networks (RNNs) to learn complex patterns from peptide sequences and their physicochemical properties. These models were trained on diverse datasets that included peptide-MHC binding affinity measurements for different MHC alleles. By extracting relevant features from the input peptide sequences, the models made accurate predictions of peptide-MHC binding affinities without relying on specific alleles. For the presentation predictions, peptides with a allele independent presentation score above 0.8 were considered probably presented, while peptides with a allele independent presentation score below 0.8 were considered less probably presented and, therefore, HLA non-binders. The percentile distribution of MHCflurry presentation scores assessed the relative ranking of peptide presentation predictions within a diverse population of peptides in MHCflurry. Factors such as peptide sequence, length, and potential MHC binding affinity were taken into account. Peptides with a presentation percentile below 1% were considered favorable presentation percentile immunopeptides, while those above 1% were considered peptides with a non-favorable presentation percentile. Additionally, inventors employed a deep learning architecture trained on a real datasets to predict proteasomal processing. Peptides with a presentation score above 0.8 were considered probably processed, while peptides with a score below 0.8 were considered less probably processed. The results from MHCflurry were visualized using the ggplot2 R package as bar plots.

To assess MHC Class I peptide antigenicity, inventors used the IEDB MHC class I peptide antigenicity prediction in Python 3.9.13. The prediction script was obtained from the Immune Epitope Database (IEDB) website at https://www.iedb.org/. The IEDB employed computational algorithms and predictive models that considered factors such as peptide length, amino acid sequence, and MHC molecule mask to estimate peptide immunogenicity. The threshold for determining immunogenic and non-immunogenic immunopeptides was set according to recommendations in the IEDB database. Peptides with an immunogenicity score below 0 were considered non-immunogenic, while peptides with a score above 0 were considered immunogenic. The results from IEDB MHC class I peptide antigenicity predictions was visualized using the ggplot2 R package as bar plots.

The invention method of tissue MHC class I immunopeptidome by antibody free approach highlights a quantitative evaluation of complex peptide mixture to reveal the diverse nature of tissue MHC class I immunopeptidome. The invention workflow comprehensively identifies at least 15671 MHC class I peptides (with iPROB < 0.99) from 2290 protein precursors in both NSCLC tissues (n=5) and healthy donors (n=5) samples. Among these, at least 8565 MHC class I peptides were quantitated (with mPROPHET qvalue < 0.01) with comparing the healthy tissue (n=5) to the NSCLC tissue (n=5) samples. Further details presents information on MHC class I peptide identification in individual tissue samples, the overlap of peptide identification between the NSCLC tissue and control tissue sample groups. Venn analysis highlights a considerably higher number of peptides in the NSCLC tissue group compared to healthy tissue (control tissue). Each search engine such as COMET (CMT), and MS FRAGGER (FRG), has inbuild algorithm therefore, to consider the comprehensiveness of data analysis. The invention employed two types of mass spectrometry data acquisition, data-dependent acquisition (DDA) and data-independent acquisition (DIA), then multi-search engine strategies for creating the spectral library for qualitative and quantitative tissue MHC class I immunopeptidome, facilitating discrimination between the NSCLC tissue and control tissue sample groups conditions. As a result, for the first time, comprehensive qualitative tissue MHC class I immunopeptidome are identified. Next step is to quality control of MHC class I immunopeptidome, the peptide length distribution in between 7-16 mer amino acids is the key feature of MHC class I peptides. In the distribution of peptide lengths for all tissue MHC class I immunopeptidomics identified peptides is shown. The most of the peptides fall into the range of 7-16 -mers and higher the enrichment of 9-mer peptides, which is consistent with the characteristic length of MHC class I immunopeptidomes.

The tissue MHC class I immunopeptidome predictions have several platforms available to date. The invention, the MHCflurry platform was implemented to provide an overview of tissue MHC class I immunopeptide enrichment among contaminant peptides in the quantitative immunopeptidomics analysis comparing non-small cell lung cancer (NSCLC) tissue to healthy tissue. MHCflurry utilized deep learning architectures, such as convolutional neural networks (CNNs) and recurrent neural networks (RNNs), to predict peptide-MHC binding affinities in an allele-independent manner. Additionally, ensemble models were used to capture allele-specific binding preferences, considering the unique characteristics and preferences of each MHC allele, including peptide anchor positions and residue preferences. Immunogenicity determination relied on parameters such as peptide length, amino acid sequence, and MHC molecule characteristics. By considering these approaches, an insight on the extent of enrichment of immunopeptides in tissue class I immunopeptidomics samples isolated NSCLC tissue and healthy control tissue was retrieved. By integrating these predictions inventors provided a comprehensive understanding of the MHC class I immunopeptide composition of tissue immunopeptidomics isolate by antibody free approach and further offering insights into the dynamics of immune recognition and peptide-MHC class I interactions in NSCLC tissue samples.

The invention approach of quantitative tissue MHC class I immunopeptidome by antibody free approach provides a insight into pattern dedicated for generation of anticancer vaccines against Non-Small Cell Lung Cancer (NSCLC), identification and isolation of NSCLC specific T cells in humans. After performing a multi-bioinformatics analysis, the invention quantitative tissue MHC class I immunopeptidome workflow reveals perturbation in immunopeptidome signaling within the NSCLC tissue samples in comparison to healthy samples group, which indicating attenuation of lymphocytes-related immunopeptidome in the NSCLC tissue samples, which might be the source of neoantigen that NSCLC tumor-specific mutations or aberrantly expressed proteins, making them attractive targets for cancer immunotherapy. However, further validation are necessary to determine the immunotherapy effectiveness. In particular, the quantitative tissue MHC class I immunopeptidomics analysis aimed to identify and characterize differences in the peptide repertoire between healthy tissue and Non-Small Cell Lung Cancer (NSCLC) tissue samples. Through comprehensive analysis using Gene Set Enrichment Analysis (GSEA), a subset of significantly changed peptides and their corresponding protein precursors belonging to the lymphocyte activation term (GO:0046649) was revealed as shown in GSEA result. The enrichment pattern suggests a decreased activity of lymphocyte-related processes in NSCLC tissue expressed by an altered immunopeptidome profile. Moreover, the human leukocyte antigen (HLA) system (the major histocompatibility complex [MHC] in humans) HLA binding evaluation of a subset of significantly changed MHC class I peptides associated with the lymphocyte activation term. Then demonstrates the differential regulation of HLA binders originating from this term in healthy tissue compared to NSCLC tissue samples. Notably, HLA binders associated with the lymphocyte activation term were more frequently upregulated in healthy tissue, indicating an increased MHC class I immunopeptide presentation. In contrast, a population of MHC class I peptides with opposing regulation was observed, but in general most of these peptides were predicted not to bind to HLA molecules. This suggests that this population of peptides contains a higher proportion of contaminants and may not be directly involved in the changed presentation patterns representing lymphocyte deactivation in NSCLC samples. The differential regulation observed in HLA binders associated with the lymphocyte activation term further supports the hypothesis of lymphocyte attenuation in NSCLC tissue samples in comparison with healthy tissue samples and further, the inventors provide a list of immunopeptide pattern dedicated for generation of anti-cancer vaccines against Non-Small Cell Lung Cancer (NSCLC), identification and isolation of NSCLC specific T cells in humans (Table 1).

Lastly, the inventors selected several unique MHC class I peptidome as the source of possible neoantigen peptidome candidates for generation of anti-cancer vaccines against Non-Small Cell Lung Cancer (NSCLC), identification and isolation of NSCLC specific T cells in humans or diagnosis, respectively. The volcano plot depicting the dysregulated protein sources and their corresponding MHC class I immunopeptides sequences derived from protein precursors of the lymphocyte activation term (GO:0046649) for diagnosing lymphocyte attenuation in Non-Small Cell Lung Cancer (NSCLC), which distinguishing NSCLC tissue from healthy tissue samples. The volcano plot specifically focuses on selected targets that are proposed in inventions, representing immunopeptides originating from protein precursors linked by the lymphocyte activation term (GO:0046649). The selected labels on the plot highlight the regulation of MHC class I immunopeptides derived from protein precursors associated with the lymphocyte activation term (GO:0046649). These labels provide important information about the upregulation and downregulation of specific MHC class I immunopeptides and their potential relevance in the context of lymphocyte activation and NSCLC samples.

Therefore, all these at least 25 quantitative immunopeptidome (Table 1), HLA binder, and the peptide length between 7-16 amino acids (majority at 9 mers) MHC class I immunopeptidome patterns for lymphocyte-related immune responses (lymphocyte attenuation) in NSCLC. Tissue MHC class I immunopeptide patterns for pattern dedicated for generation of anti-cancer vaccines against Non-Small Cell Lung Cancer (NSCLC), identification and isolation of NSCLC specific T cells in humans and differential immunopeptides of NSCLC from healthy tissues that is a list of the following 25 quantitative MHC class I immunopeptide Lys-Pro-Ala-Gly-Pro-Pro-Gly-Ile-Leu-Ala-Leu, Tyr-Glu-Phe-Lys-Phe-Pro-Asn-Arg-Leu, Asp-Val-Tyr-Asn-His-Phe-Leu-Leu-Tyr, Phe-Thr-Leu-Gln-Ser-Glu-Leu, Lys-Glu-Asp-Asp-Ala-Pro-Arg-Thr-Ile, Thr-Glu-Phe-Thr-Pro-Thr-Glu-Lys, Phe-Arg-Ile-Gly-Phe-Gly-Ser-Phe, Lys-Thr-Asn-Gly-Lys-Phe-Leu-Ile-Arg, Met-Pro-Val-Gly-Pro-Asp-Ala-Ile-Leu-Arg-Tyr, Gln-Gly-Phe-Pro-Asn-Arg-Val-Val-Phe, Tyr-Val-Tyr-Glu-Tyr-Pro-Ser-Arg-Tyr, Asn-Ala-Ala-Glu-Arg-Arg-Gly-Pro-Leu, Ala-Pro-Arg-Thr-Val-Ala-Leu-Thr-Ala, Met-Pro-Val-Gly-Pro-Asp-Ala-Ile-Leu-Arg-Tyr, Thr-Ile-Ile-Asp-Ile-Leu-Thr-Lys-Arg, Arg-Tyr-Leu-Glu-Lys-Pro-Met-Glu-Ile, Gly-Thr-Tyr-Val-Ser-Ser-Val-Pro-Arg, Gly-Thr-Asp-Pro-Thr-Pro-Gln-His-Tyr, Ala-Ile-Leu-Asp-Glu-Thr-Lys-Gly-Asp-Tyr, Val-Leu-Lys-Thr-Pro-Ser-Ala-Ala-Tyr, Asp-Leu-Val-Pro-Val-Pro-Thr-Asn-Leu, Val-Ile-Glu-Glu-Val-Pro-Gly-Glu-Leu, Tyr-Gln-Lys-Met-Tyr-Gly-Ile-Ser-Leu, Lys-Gly-Asp-Gly-Pro-Val-Gln-Gly-Ile-Ile-Asn-Phe, Ile-Glu-Val-Asp-Asp-Glu-Arg-Lys-Leu-Arg-Thr-Phe wherein the MHC class I immunopeptides level of mentioned 25 peptides are up or down regulated between the comparison of healthy tissue with matched NSCLC patient-derived tissue samples.

The tissue MHC class I immunopeptide patterns pattern, wherein healthy tissue MHC class I immunopeptide levels of the following 18 quantitative peptides Lys-Pro-Ala-Gly-Pro-Pro-Gly-Ile-Leu-Ala-Leu, Tyr-Glu-Phe-Lys-Phe-Pro-Asn-Arg-Leu, Asp-Val-Tyr-Asn-His-Phe-Leu-Leu-Tyr, Phe-Thr-Leu-Gln-Ser-Glu-Leu, Lys-Glu-Asp-Asp-Ala-Pro-Arg-Thr-Ile, Thr-Glu-Phe-Thr-Pro-Thr-Glu-Lys, Phe-Arg-Ile-Gly-Phe-Gly-Ser-Phe, Lys-Thr-Asn-Gly-Lys-Phe-Leu-Ile-Arg, Met-Pro-Val-Gly-Pro-Asp-Ala-Ile-Leu-Arg-Tyr, Gln-Gly-Phe-Pro-Asn-Arg-Val-Val-Phe, Tyr-Val-Tyr-Glu-Tyr-Pro-Ser-Arg-Tyr, Asn-Ala-Ala-Glu-Arg-Arg-Gly-Pro-Leu, Ala-Pro-Arg-Thr-Val-Ala-Leu-Thr-Ala, Met-Pro-Val-Gly-Pro-Asp-Ala-Ile-Leu-Arg-Tyr, Thr-Ile-Ile-Asp-Ile-Leu-Thr-Lys-Arg, Arg-Tyr-Leu-Glu-Lys-Pro-Met-Glu-Ile, Gly-Thr-Tyr-Val-Ser-Ser-Val-Pro-Arg, Gly-Thr-Asp-Pro-Thr-Pro-Gln-His-Tyr wherein the tissue MHC class I immunopeptide level of mentioned 18 immunopeptides are up-regulated in healthy tissue with matched NSCLC patient-derived tissue samples.

The tissue MHC class I immunopeptide patterns pattern, wherein healthy tissue MHC class I immunopeptide levels of the following 7 quantitative peptides Ala-Ile-Leu-Asp-Glu-Thr-Lys-Gly-Asp-Tyr, Val-Leu-Lys-Thr-Pro-Ser-Ala-Ala-Tyr, Asp-Leu-Val-Pro-Val-Pro-Thr-Asn-Leu, Val-Ile-Glu-Glu-Val-Pro-Gly-Glu-Leu, Tyr-Gln-Lys-Met-Tyr-Gly-Ile-Ser-Leu, Lys-Gly-Asp-Gly-Pro-Val-Gln-Gly-Ile-Ile-Asn-Phe, Ile-Glu-Val-Asp-Asp-Glu-Arg-Lys-Leu-Arg-Thr-Phe wherein the tissue MHC class I immunopeptide level of mentioned 7 immunopeptides are down-regulated in healthy tissue with matched NSCLC patient-derived tissue samples.

**Table 1. MHC class I quantitative tissue immunopeptides therapeutic panel for NSCLC. The sequence of therapeutic MHC class I peptides, source protein identifier for each peptide, length, as well as HLA binding prediction evaluation and fold changes of peptides either up-regulated (↑), down-regulated (↓) in comparison of healthy tissue to non-small cell lung cancer (NSCLC) tissue samples. The up and down regulated refers only to MHC class I tissue quantitative peptides; it shows how many times more signaling intensities present in the tissue of healthy samples were higher than in non-small cell lung cancer (NSCLC) tissue samples.**

| Source protein | Peptide | Length | HLA binding evaluation | Regulation (control vs. NSCLC tumor) |
|---|---|---|---|---|
| MYH9_HUMAN | Lys-Pro-Ala-Gly-Pro-Pro- Gly-Ile-Leu-Ala-Leu | 11 | Binder | ↑ |
| LEG1_HUMAN | Tyr-Glu-Phe-Lys-Phe-Pro- Asn-Arg-Leu | 9 | Binder | ↑ |
| SPT6H_HUMAN | Asp-Val-Tyr-Asn-His-Phe- Leu-Leu-Tyr | 9 | Binder | ↑ |
| RAGE_HUMAN | Phe-Thr-Leu-Gln-Ser-Glu- Leu | 7 | Binder | ↑ |
| TYY1_HUMAN | Lys-Glu-Asp-Asp-Ala-Pro- Arg-Thr-Ile | 9 | Binder | ↑ |
| B2MG_HUMAN | Thr-Glu-Phe-Thr-Pro-Thr- Glu-Lys | 8 | Binder | ↑ |
| ITB1_HUMAN | Phe-Arg-Ile-Gly-Phe-Gly- Ser-Phe | 8 | Binder | ↑ |
| KSYK_HUMAN | Lys-Thr-Asn-Gly-Lys-Phe- Leu-Ile-Arg | 9 | Binder | ↑ |
| BAG6_HUMAN | Met-Pro-Val-Gly-Pro-Asp- Ala-Ile-Leu-Arg-Tyr | 11 | Binder | ↑ |
| MYH9_HUMAN | Gln-Gly-Phe-Pro-Asn-Arg- Val-Val-Phe | 9 | Binder | ↑ |
| CASL_HUMAN | Tyr-Val-Tyr-Glu-Tyr-Pro- Ser-Arg-Tyr | 9 | Binder | ↑ |
| DRA_HUMAN | Asn-Ala-Ala-Glu-Arg-Arg- Gly-Pro-Leu | 9 | Binder | ↑ |
| DPB1_HUMAN | Ala-Pro-Arg-Thr-Val-Ala- Leu-Thr-Ala | 9 | Binder | ↑ |
| BAG6_HUMAN | Met-Pro-Val-Gly-Pro-Asp- Ala-Ile-Leu-Arg-Tyr | 11 | Binder | ↑ |
| ANXA1_HUMAN | Thr-Ile-Ile-Asp-Ile-Leu-Thr- Lys-Arg | 9 | Binder | ↑ |
| STAT3_HUMAN | Arg-Tyr-Leu-Glu-Lys-Pro- Met-Glu-Ile | 9 | Binder | ↑ |
| DOA_HUMAN | Gly-Thr-Tyr-Val-Ser-Ser- Val-Pro-Arg | 9 | Binder | ↑ |
| FYN_HUMAN | Gly-Thr-Asp-Pro-Thr-Pro- Gln-His-Tyr | 9 | Binder | ↑ |
| ANXA1_HUMAN | Ala-Ile-Leu-Asp-Glu-Thr- Lys-Gly-Asp-Tyr | 10 | Binder | ↓ |
| GELS_HUMAN | Val-Leu-Lys-Thr-Pro-Ser- Ala-Ala-Tyr | 9 | Binder | ↓ |
| GELS_HUMAN | Asp-Leu-Val-Pro-Val-Pro- Thr-Asn-Leu | 9 | Binder | ↓ |
| GELS_HUMAN | Val-Ile-Glu-Glu-Val-Pro- Gly-Glu-Leu | 9 | Binder | ↓ |
| ANXA1_HUMAN | Tyr-Gln-Lys-Met-Tyr-Gly- Ile-Ser-Leu | 9 | Binder | ↓ |
| SODC_HUMAN | Lys-Gly-Asp-Gly-Pro-Val- Gln-Gly-Ile-Ile-Asn-Phe | 12 | Binder | ↓ |
| RS6_HUMAN | Ile-Glu-Val-Asp-Asp-Glu- Arg-Lys-Leu-Arg-Thr-Phe | 12 | Binder | ↓ |

These at least 25 quantitative immunopeptidome presented in table 1 disclosing MHC class I immunopeptide sequences derived from protein precursors of the lymphocyte activation term for diagnosing lymphocyte attenuation in Non-Small Cell Lung Cancer (NSCLC) tissue samples.

### References

[1] A. W. Purcell, S. H. Ramarathinam, and N. Ternette, "Mass spectrometry-based identification of MHC-bound peptides for immunopeptidomics," Nat. Protoc., vol. 14, no. 6, pp. 1687-1707, Jun. 2019, doi: 10.1038/s41596-019-0133-y.
[2] F.-R. Schumacher et al., "Building proteomic tool boxes to monitor MHC class I and class II peptides," Proteomics, vol. 17, no. 1-2, Jan. 2017, doi: 10.1002/pmic.201600061.
[3] M. Bassani-Sternberg, S. Pletscher-Frankild, L. J. Jensen, and M. Mann, "Mass spectrometry of human leukocyte antigen class I peptidomes reveals strong effects of protein abundance and turnover on antigen presentation," Mol. Cell. Proteomics MCP, vol. 14, no. 3, pp. 658-673, Mar. 2015, doi: 10.1074/mcp.M114.042812.
[4] D. Gebreselassie, H. Spiegel, and S. Vukmanovic, "Sampling of major histocompatibility complex class I-associated peptidome suggests relatively looser global association of HLA-B∗5101 with peptides," Hum. Immunol., vol. 67, no. 11, pp. 894-906, Nov. 2006, doi: 10.1016/j.humimm.2006.08.294.

## Claims

1. Tissue MHC class I immunopeptide pattern dedicated for generation of anti-cancer vaccines against Non-Small Cell Lung Cancer (NSCLC), identification and isolation of NSCLC specific T cells in humans that is a list of the following 25 quantitative MHC class I immunopeptide Lys-Pro-Ala-Gly-Pro-Pro-Gly-Ile-Leu-Ala-Leu, Tyr-Glu-Phe-Lys-Phe-Pro-Asn-Arg-Leu, Asp-Val-Tyr-Asn-His-Phe-Leu-Leu-Tyr, Phe-Thr-Leu-Gln-Ser-Glu-Leu, Lys-Glu-Asp-Asp-Ala-Pro-Arg-Thr-Ile, Thr-Glu-Phe-Thr-Pro-Thr-Glu-Lys, Phe-Arg-Ile-Gly-Phe-Gly-Ser-Phe, Lys-Thr-Asn-Gly-Lys-Phe-Leu-Ile-Arg, Met-Pro-Val-Gly-Pro-Asp-Ala-Ile-Leu-Arg-Tyr, Gln-Gly-Phe-Pro-Asn-Arg-Val-Val-Phe, Tyr-Val-Tyr-Glu-Tyr-Pro-Ser-Arg-Tyr, Asn-Ala-Ala-Glu-Arg-Arg-Gly-Pro-Leu, Ala-Pro-Arg-Thr-Val-Ala-Leu-Thr-Ala, Met-Pro-Val-Gly-Pro-Asp-Ala-Ile-Leu-Arg-Tyr, Thr-Ile-Ile-Asp-Ile-Leu-Thr-Lys-Arg, Arg-Tyr-Leu-Glu-Lys-Pro-Met-Glu-Ile, Gly-Thr-Tyr-Val-Ser-Ser-Val-Pro-Arg, Gly-Thr-Asp-Pro-Thr-Pro-Gln-His-Tyr, Ala-Ile-Leu-Asp-Glu-Thr-Lys-Gly-Asp-Tyr, Val-Leu-Lys-Thr-Pro-Ser-Ala-Ala-Tyr, Asp-Leu-Val-Pro-Val-Pro-Thr-Asn-Leu, Val-Ile-Glu-Glu-Val-Pro-Gly-Glu-Leu, Tyr-Gln-Lys-Met-Tyr-Gly-Ile-Ser-Leu, Lys-Gly-Asp-Gly-Pro-Val-Gln-Gly-Ile-Ile-Asn-Phe, Ile-Glu-Val-Asp-Asp-Glu-Arg-Lys-Leu-Arg-Thr-Phe wherein the MHC class I immunopeptides level of mentioned 25 peptides are up or down regulated between the comparison of healthy tissue with matched NSCLC patient-derived tissue samples.

2. The tissue MHC class I immunopeptide pattern according to claim 1, wherein healthy tissue MHC class I immunopeptide levels of the following 18 quantitative peptides Lys-Pro-Ala-Gly-Pro-Pro-Gly-Ile-Leu-Ala-Leu, Tyr-Glu-Phe-Lys-Phe-Pro-Asn-Arg-Leu, Asp-Val-Tyr-Asn-His-Phe-Leu-Leu-Tyr, Phe-Thr-Leu-Gln-Ser-Glu-Leu, Lys-Glu-Asp-Asp-Ala-Pro-Arg-Thr-Ile, Thr-Glu-Phe-Thr-Pro-Thr-Glu-Lys, Phe-Arg-Ile-Gly-Phe-Gly-Ser-Phe, Lys-Thr-Asn-Gly-Lys-Phe-Leu-Ile-Arg, Met-Pro-Val-Gly-Pro-Asp-Ala-Ile-Leu-Arg-Tyr, Gln-Gly-Phe-Pro-Asn-Arg-Val-Val-Phe, Tyr-Val-Tyr-Glu-Tyr-Pro-Ser-Arg-Tyr, Asn-Ala-Ala-Glu-Arg-Arg-Gly-Pro-Leu, Ala-Pro-Arg-Thr-Val-Ala-Leu-Thr-Ala, Met-Pro-Val-Gly-Pro-Asp-Ala-Ile-Leu-Arg-Tyr, Thr-Ile-Ile-Asp-Ile-Leu-Thr-Lys-Arg, Arg-Tyr-Leu-Glu-Lys-Pro-Met-Glu-Ile, Gly-Thr-Tyr-Val-Ser-Ser-Val-Pro-Arg, Gly-Thr-Asp-Pro-Thr-Pro-Gln-His-Tyr wherein the tissue MHC class I immunopeptide level of mentioned 18 immunopeptides are up-regulated in healthy tissue with matched NSCLC patient-derived tissue samples.

3. The tissue MHC class I immunopeptide pattern according to claim 1, wherein healthy tissue MHC class I immunopeptide levels of the following 7 quantitative peptides Ala-Ile-Leu-Asp-Glu-Thr-Lys-Gly-Asp-Tyr, Val-Leu-Lys-Thr-Pro-Ser-Ala-Ala-Tyr, Asp-Leu-Val-Pro-Val-Pro-Thr-Asn-Leu, Val-Ile-Glu-Glu-Val-Pro-Gly-Glu-Leu, Tyr-Gln-Lys-Met-Tyr-Gly-Ile-Ser-Leu, Lys-Gly-Asp-Gly-Pro-Val-Gln-Gly-Ile-Ile-Asn-Phe, Ile-Glu-Val-Asp-Asp-Glu-Arg-Lys-Leu-Arg-Thr-Phe wherein the tissue MHC class I immunopeptide level of mentioned 7 immunopeptides are down-regulated in healthy tissue with matched NSCLC patient-derived tissue samples.

4. The NSCLC tissue MHC class I immunopeptide pattern according to claims 1-4, wherein the NSCLC tissue peptides are defined with tandem mass spectrometry.


SEQUENCE LISTING

<110> Uniwersytet Gdanski

<120> Tissue MHC class I immunopeptide pattern dedicated for generation of anti-cancer
vaccines against Non-Small Cell Lung Cancer (NSCLC), identification and isolation of NSCLC specific
T cells in humans

<130>

<140>

<141>

<160> 25

<212> PRT

<213> Homo sapiens

<211> 11

<212> PRT

<213> Homo sapiens

<210> 1

<400> 1

Lys Pro Ala Gly Pro Pro Gly Ile Leu Ala Leu

                5                   10

<211> 9

<212> PRT

<213> Homo sapiens

<210> 2

<400> 2

Tyr Glu Phe Lys Phe Pro Asn Arg Leu
                5

<211> 9
<212> PRT
<213> Homo sapiens
<210> 3
<400> 3

Asp Val Tyr Asn His Phe Leu Leu Tyr
                5

<211> 7
<212> PRT
<213> Homo sapiens
<210> 4
<400> 4

Phe Thr Leu Gln Ser Glu Leu
                5

<211> 9
<212> PRT
<213> Homo sapiens

<210> 5

<400> 5

Lys Glu Asp Asp Ala Pro Arg Thr Ile

                5

<211> 8

<212> PRT

<213> Homo sapiens

<210> 6

<400> 6

Thr Glu Phe Thr Pro Thr Glu Lys

                5

<211> 8

<212> PRT

<213> Homo sapiens

<210> 7

<400> 7

Phe Arg Ile Gly Phe Gly Ser Phe

                5

<211> 9

<212> PRT

<213> Homo sapiens

<210> 8

<400> 8

Lys Thr Asn Gly Lys Phe Leu Ile Arg

                5

<211> 11

<212> PRT

<213> Homo sapiens

<210> 9

<400> 9

Met Pro Val Gly Pro Asp Ala Ile Leu Arg Tyr

                5                   10

<211> 9

<212> PRT

<213> Homo sapiens

<210> 10

<400> 10

Gln Gly Phe Pro Asn Arg Val Val Phe

                5

<211> 9

<212> PRT

<213> Homo sapiens

<210> 11

<400> 11

Tyr Val Tyr Glu Tyr Pro Ser Arg Tyr

                5

<211> 9

<212> PRT

<213> Homo sapiens

<210> 12

<400> 12

Asn Ala Ala Glu Arg Arg Gly Pro Leu

                5

<211> 9

<212> PRT

<213> Homo sapiens

<210> 13

<400> 13

Ala Pro Arg Thr Val Ala Leu Thr Ala

                5

<211> 11

<212> PRT

<213> Homo sapiens

<210> 14

<400> 14

Met Pro Val Gly Pro Asp Ala Ile Leu Arg Tyr

                5                   10

<211> 9

<212> PRT

<213> Homo sapiens

<210> 15

<400> 15

Thr Ile Ile Asp Ile Leu Thr Lys Arg

                5

<211> 9

<212> PRT
<213> Homo sapiens
<210> 16
<400> 16
Arg Tyr Leu Glu Lys Pro Met Glu Ile
                5
<211> 9
<212> PRT
<213> Homo sapiens
<210> 17
<400> 17
Gly Thr Tyr Val Ser Ser Val Pro Arg
                5
<211> 9
<212> PRT
<213> Homo sapiens
<210> 18
<400> 18
Gly Thr Asp Pro Thr Pro Gln His Tyr
                5

<211> 10

<212> PRT

<213> Homo sapiens

<210> 19

<400> 19

Ala Ile Leu Asp Glu Thr Lys Gly Asp Tyr

                5                   10

<211> 9

<212> PRT

<213> Homo sapiens

<210> 20

<400> 20

Val Leu Lys Thr Pro Ser Ala Ala Tyr

                5

<211> 9

<212> PRT

<213> Homo sapiens

<210> 21

<400> 21

Asp Leu Val Pro Val Pro Thr Asn Leu
                5
<211> 9
<212> PRT
<213> Homo sapiens
<210> 22
<400> 22
Val Ile Glu Glu Val Pro Gly Glu Leu
                5
<211> 9
<212> PRT
<213> Homo sapiens
<210> 23
<400> 23
Tyr Gln Lys Met Tyr Gly Ile Ser Leu
                5
<211> 12
<212> PRT

<213> Homo sapiens

<210> 24

<400> 24

Lys Gly Asp Gly Pro Val Gln Gly Ile Ile Asn Phe

                5                   10

<211> 12

<212> PRT

<213> Homo sapiens

<210> 25

<400> 25

Ile Glu Val Asp Asp Glu Arg Lys Leu Arg Thr Phe

                5                   10